# EUROPÄISCHE PATENTSCHRIFT

(11) **EP 1 363 542 B1**
(45) Veröffentlichungstag und Bekanntmachung des Hinweises auf die Patenterteilung: **31.10.2007**
(21) Anmeldenummer: 02703605.2
(22) Anmeldetag: 27.02.2002
(51) Int. Cl.: A61B 17/32

(54) **CHIRURGISCHES INSTRUMENT**
SURGICAL INSTRUMENT
INSTRUMENT CHIRURGICAL

(30) Priorität: 01.03.2001 DE 20103630 U
(43) Veröffentlichungstag der Anmeldung: 26.11.2003
(73) Patentinhaber: Widmann, Heinrich, 88637 Buchheim (DE)
(72) Erfinder: Widmann, Heinrich, 88637 Buchheim (DE)
(74) Vertreter: Daub, Thomas
(86) Internationale Anmeldenummer: PCT/EP2002/002107
(87) Internationale Veröffentlichungsnummer: WO 2002/069815

(56) Entgegenhaltungen:
- EP-A- 0 838 198
- WO-A-95/05123
- DE-A- 19 713 067
- DE-U- 20 002 940
- US-A- 5 827 263

## Beschreibung

Die Erfindung betrifft ein chirurgisches Instrument mit einem Linearelement und einem in oder an dem Linearelement gleitbar und lösbar angeordneten Ein- oder Ansatz, wobei der Ein- oder Ansatz bzw. ein diesem zugeordnetes Führungselement auf zumindest einer Führungsbahn gegenüber dem Linearelement geführt ist und wobei in bzw. aus der Führungsbahn ein Anschlag hinter den Ein- oder Ansatz bzw. das Führungselement bewegbar ist.

Die vorliegende Erfindung soll sich auf alle möglichen Arten von chirurgischen Instrumenten beziehen beispielsweise auch auf Rohrschaftinstrumente. Insbesondere bezieht sie sich aber auf Schiebeschaftinstrumente. Mit ihnen werden schneidende, scherende, klemmende od.dgl. Eingriffe, beispielsweise im menschlichen Körper, vorgenommen. Hierbei wird über entsprechende Handgriffe der Schieber auf einer Gleitfläche des Schaftes bewegt und an dessen Ende in der Regel ein Maul betätigt.

Vor allem finden derartige Schiebeschaftinstrument als Knochenstanzen, Ohrzangen und in der gynäkologischen Biopsie Anwendung. Dabei ist ein generelles Problem, dass es sich bei derartigen chirurgischen Instrumenten um Produkte mit verschiedensten Gelenken, Führungen oder Nuten und Rinnen handelt. Derartige Instrumente sind deshalb ausserordentlich schwer zu reinigen und zu sterilisieren, wobei aber gerade die Reinigung chirurgischer Instrumente äusserst wichtig ist. Die Hygierieanforderungen für solche chirurgische Instrumente steigen durch die Gefahr der Übertragung von Krankheiten, beispielsweise von Hepatitis oder Aids, stark an.

Ein Schiebeschaftinstrument ist beispielsweise aus der DE 43 16 769 C1 bekannt. Bei diesem Instrument besteht allerdings die Gefahr, dass sich der Schieber ungewollt vom Schaft löst.

Aus der EP 0 838 198 A ist ein chirurgisches Instrument bekannt, bei dem zum Bewegen von Maulteilen in einem Aussenrohr eine Zug- bzw. Druckstange geführt ist. Diese Stange besitzt zwei Ausnehmungen, wobei in eine Ausnehmung eine Schraube und in eine andere Ausnehmung eine Rastnase eines Zangenschenkels eingreift. Zum Freigeben der Stange muss die Schraube von Hand gelöst werden.

Aus der DE 197 13 067 A ist ein Arthroskopie-Instrument bekannt, bei dem ebenfalls in einem Aussenrohr eine Zug- bzw. Druckstange geführt wird. Diese besitzt im Bereich des Handgriffs eine Mulde. Zum Festlegen der Stangen in dem Handstück bzw. zum Freigeben der Stange dient ein Drehbolzen mit einem hälftigen Einstich. Zum Lösen bzw. Festlegen muss der Drehbolzen jeweils um 180° gedreht werden.

Ein chirurgisches Instrument der oben genannten Art ist aus der WO 95/05123 bekannt. Dabei ist dort ein von einem Schieber abragender Stift in einem Schlitz eines Schiebeschaftelementes geführt. In diesen Schlitz greift ein Druckknopf ein, der gegen die Kraft einer Feder bewegbar ist. Der Druckknopf weist in dem Schlitz einen Anschlag auf, der die Bewegung des Stiftes und damit des Schiebers begrenzt. Durch Druck auf den Druckknopf wird dieser Anschlag aus der lichten Weite des Schlitzes herausbewegt, so dass der Schieber weiter nach hinten geführt werden kann, so dass auch eine T-Schiene ausser Eingriff mit einer entsprechend geformten Nut des Schaftelementes gelangt und der Schieber vom Schaftelement abgenommen werden kann. Beim Zusammenbau des Instrumentes muss der Druckknopf wiederum betätigt werden, damit der Stift hinter den Anschlag gelangt.

Der vorliegenden Erfindung liegt die Aufgabe zugrunde, ein Instrument der oben genannten Art zu entwickeln, bei welchem ein ungewolltes Lösen des Ein- oder Ansatzes vom Linearelement wirksam vermieden wird, gleichzeitig aber ein leichtes Auseinandernehmen und Zusammenbauen gewährleistet ist.

Zur Lösung dieser Aufgabe führt, dass der Anschlag schräg in einem stumpfen Winkel, bevorzugt von unten her, zu der Führungsbahn geführt ist.

Durch diese Ausgestaltung wird vermieden, dass bspw. bei einem Schiebeschaftinstrument der Schieber bzw. seine Führungselemente unbeabsichtigt aus einer Längsnut herausgleiten, und dass sich somit Schieber vom Schaft unbeabsichtigt trennt.

Der Anschlag ist bevorzugt in einem Sackloch in dem Schaft geführt und greift von unten her in die lichte Weite der Führungsbahn, bspw. eine Längsnut ein. Dabei ist das Sackloch schräg zur Nut angestellt, so dass auch der als Druckstift ausgebildete Anschlag das Führungselement von hinten schräg abstützt. Hierdurch ist die Abstützung des Führungselementes verbessert.

In dem Sackloch selbst stützt sich der Druckstift wiederum gegen einen Kraftspeicher, bevorzugt gegen eine Schraubenfeder, ab. Das bedeutet, dass der Druckstift, wenn nicht seine Lage bewusst geändert wird, in Verriegelungslage gehalten wird. Erst durch ein bewusstes Führen des Druckstiftes gegen die Kraft der Schraubenfeder kann der Druckstift aus der Verriegelungslage geführt werden, wozu ein Schieberknopf aussen am Schaft vorgesehen ist. Der Schieberknopf ist über einen Verbindungsstift mit dem Druckstift verbunden, wobei zum leichteren Zusammenbau der Druckstift mit einem Gewindeschaft in eine Gewindebohrung in dem Druckstift eingeschraubt ist. Der Schieberknopf selbst wird in einer Rinne aussen am Schaft geführt.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine Draufsicht auf ein erfindungsgemässes Schiebeschaftinstrument;
Figur 2 eine Draufsicht auf Teile des Schiebeschaftinstrumentes gemäss Figur 1;
Figur 3 eine Draufsicht auf Teile des Schiebeschaftinstrumentes gemäss Figur 1, teilweise aufgebrochen;
Figur 4 Draufsichten auf Teile eines Verriegelungsmechanismusses für das Schiebeschaftinstrument gemäss Figur 1.

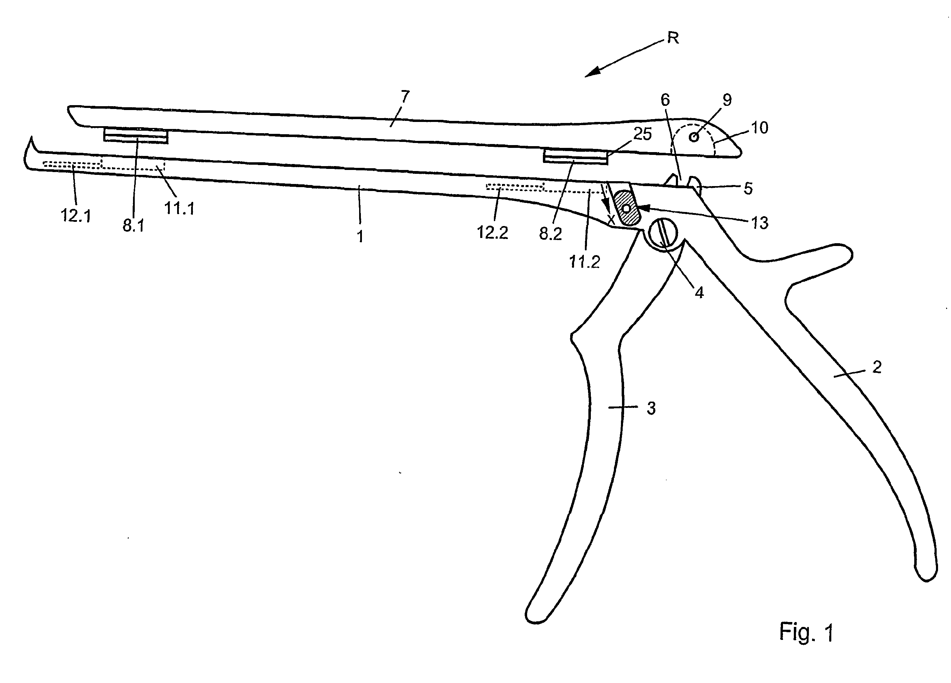

Gemäss Figur 1 weist ein Schiebeschaftinstrument R einen Schaft 1 auf, der einstückig in einen Handgriff 2 übergeht. Zwischen dem Schaft 1 und dem Handgriff 2 ist ein weiterer Handgriff 3 eingesetzt und gelenkig über eine Schraube 4 mit dem Schaft 1 verbunden. Der Handgriff 3 durchsetzt den Schaft 1 und schaut andererseits aus dem Schaft 1 mit einem Ende 5 heraus, wobei in das Ende 5 ein Schlitz 6 eingeformt ist.

Dem Schaft 1 ist ein Schieber 7 zugeordnet, welcher zwei Führungselemente 8.1 und 8.2 besitzt. Ferner besitzt der Schieber 7 einends einen Querstift 9, welcher eine gestrichelt angedeutete Ausnehmung 10 durchsetzt. Diese Ausnehmung 10 dient beim Zusammensetzen des Schiebeschaftinstrumentes der Aufnahme des Endes 5 des Handgriffes 3, wobei der Querstift 9 in den Schlitz 6 eingleitet. Gleichzeitig greifen auch die Führungselemente 8.1 und 8.2 in Nuten 11.1 und 11.2 in dem Schaft 1 ein und können anschliessend in Längsnuten 12.1 und 12.2. geführt werden.

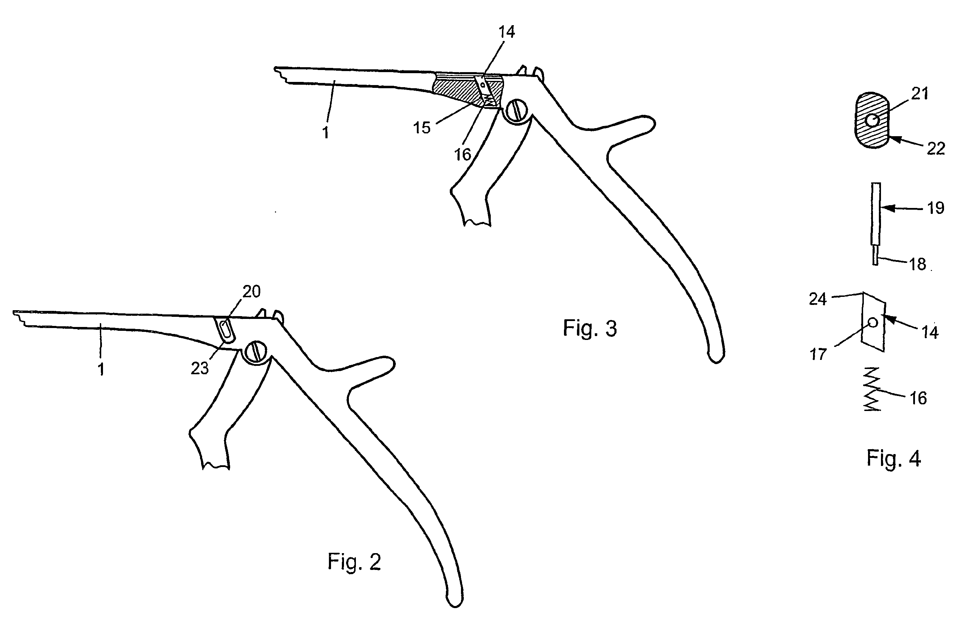

Erfindungsgemäss ist zumindest der Nut 11.2 hinter dem Führungselement 8.2 ein Verriegelungsmechanismus 13 zugeordnet. Dieser Verriegelungsmechanismus 13 besteht gemäss Figur 4 aus vier Teilen. Die eigentliche Verriegelung wird durch einen Druckstift 14 bewirkt, der in Gebrauchslage gemäss Figur 3 in einem Sackloch 15, welches in den Schaft 1 eingeformt ist, gleitet. In dem Sackloch 15 stützt sich der Druckstift 14 gegen eine Feder 16 ab. Die Feder 16 versucht, den Druckstift 14 aus dem Sackloch 15 herauszudrängen, so dass der Druckstift 14 in die lichte Weite der Nut 11.2 eingreift.

Der Druckstift 14 weist ferner eine Gewindebohrung 17 auf, in welche ein Gewindeabschnitt 18 eines Verbindungsstiftes 19 eingesetzt werden kann. In Gebrauchslage durchsetzt dieser Verbindungsstift 19 ein Langloch 20 in dem Schaft 1 (siehe Figur 2).

Nach dem Durchsetzen des Langloches 20 greift der Verbindungsstift 19 in eine Bohrung 21 eines Schieberknopfes 22 ein und ist mit diesem Schieberknopf 22 verbunden. Der Schieberknopf 22 wird in einer Rinne 23 in dem Schaft 1 geführt, wobei die Rinne 23 dem Schaft 1 von aussen her eingeformt ist.

### Die Funktionsweise der vorliegenden Erfindung ist folgende:

Beim Zusammensetzen des erfindungsgemässen chirurgischen Instrumentes R wird der Schieber 7 auf den Schaft 1 aufgelegt, wobei der Querstift 9 in den Schlitz 6 eingleitet. Das Führungselement 8.1 greift in die Nut 11.1 und das Führungselement 8.2 in die Nut 11.2 ein, wobei der Druckstift 14 gegen die Kraft der Feder 16 in das Sackloch 15 verdrängt wird. Sobald das Führungselement 8.2 den Grund der Nut 11.2 erreicht hat, schnappt der Druckstift 14 hinter das Führungselement 8.2, da eine Steuerkante 24 infolge der Schrägstellung des Sackloches 15 die hintere Kante 25 des Führungselementes 8.2 erreicht hat. In diesem Augenblick ist das Führungselement 8.2 bereits in geringem Umfang in die Längsnut 12.2 eingeglitten, so dass das T-förmige Führungselement 8.2 in der Längsnut 12.2 gefangen ist. Die Längsnut 12.2 ist querschnittlich ebenfalls T-förmig ausgebildet und stellt so für das Führungselement 8.2 eine hinterschnittene Nut dar.

Nunmehr kann das Instrument gebraucht werden, der Schieber 7 ist nicht vom Schaft zu lösen.

Um jedoch den Schieber 7 vom Schaft 1 zu lösen, wird der Schieberknopf 22 in Richtung x betätigt, wodurch der Druckstift 14 in das Sackloch 15 gegen die Kraft der Feder 16 versenkt werden kann. Dadurch wird die lichte Weite der Nut 11.2 wieder frei gegeben, so dass das Führungselement 8.2 aus der Längsnut 12.2 heraus gleiten kann und aus der Nut 11.2 entnommen werden kann. In diesem Augenblick kann auch das Führungselement 8.1 aus der Nut 11.1 und der Querstift 9 aus dem Schlitz 6 herausgenommen werden.

## Patentansprüche

1. Chirurgisches Instrument mit einem Linearelement (1) und einem in oder an dem Linearelement (1) gleitbar und lösbar angeordneten Ein- oder Ansatz (7), wobei der Ein- oder Ansatz (7) bzw. ein diesem zugeordnetes Führungselement (8.1, 8.2) auf zumindest einer Führungsbahn (11.1, 11.2; 12.1 12.2) gegenüber dem Linearelement (1) geführt ist und wobei in bzw. aus der Führungsbahn (11.2, 12.2) ein Anschlag (14) hinter den Ein- oder Ansatz (7) bzw. das Führungselement (B.2) bewegbar ist,
**dadurch gekennzeichnet,**
**dass** der Anschlag (14) schräg in einem stumpfen Winkel, bevorzugt von unten her, zu der Führungsbahn (11.2) geführt ist.

2. Chirurgisches Instrument nach Anspruch 1, **dadurch gekennzeichnet, dass** der Anschlag (14) in einem Sackloch (15) in dem Linearelement (1) geführt ist.

3. Chirurgisches Instrument nach Anspruch 1 oder 2, **dadurch gekennzeichnet, dass** sich der Anschlag (14) gegen einen Kraftspeicher (16) abstützt.

4. Chirurgisches Instrument nach Anspruch 3, **dadurch gekennzeichnet, dass** der Kraftspeicher eine Schraubenfeder (16) ist.

5. Chirurgisches Instrument nach wenigstens einem der Ansprüche 1 bis 4, **dadurch gekennzeichnet, dass** der Anschlag (14) über einen Verbindungsstift (19) mit einem Schieberknopf (22) verbunden ist, der sich aussen am Schaft (1) befindet.

6. Chirurgisches Instrument nach Anspruch 5, **dadurch gekennzeichnet, dass** der Verbindungsstift (19) einen Gewindeabschnitt (18) aufweist, mit dem er in eine Gewindebohrung (17) in dem Anschlag einsetzbar ist.

7. Chirurgisches Instrument nach Anspruch 5 oder 6, **dadurch gekennzeichnet, dass** der Schieberknopf (22) in einer Rinne (23) im Linearelement (1) geführt ist.

8. Chirurgisches Instrument nach wenigstens einem der Ansprüche 1 bis 7, **dadurch gekennzeichnet, dass** das Linearelement ein Schaft (1) und der Ein- oder Ansatz ein Schieber (7) eines Schiebeschaftinstrumentes ist, wobei der Schaft (1) eine Längsnut (11.1, 11.2; 12.1, 12.2) als Führung aufweist..

9. Chirurgisches Instrument nach wenigstens einem der Ansprüche 1 bis 8, **dadurch gekennzeichnet, dass** als Anschlag ein Druckstift (14) vorgesehen ist.

## Claims

1. Surgical instrument with a linear element (1) and with an insert or attachment (7) slidably and detachably disposed in or on the linear element (1), said insert or attachment (7) and/or guide element (8.1, 8.2) associated therewith being guided relative to the linear element (1) on at least one guide track (11.1, 11.2; 12.1, 12.2), and a limit stop (14) being movable into or out of the guide track (11.2, 12.2) behind the insert or attachment (7) and/or the guide element (8.2), **characterized in that** the limit stop (14) is guided obliquely at an obtuse angle to the guide track (11.2), preferably from below.

2. Surgical instrument according to Claim 1, **characterized in that** the limit stop (14) is guided in a blind hole (15) in the linear element (1).

3. Surgical instrument according to Claim 1 or 2, **characterized in that** the limit stop (14) bears against an energy accumulator (16).

4. Surgical instrument according to Claim 3, **characterized in that** the energy accumulator is a helical spring (16).

5. Surgical instrument according to at least one of Claims 1 to 4, **characterized in that** the limit stop (14) is connected via a connection pin (19) to a slide button (22) located on the outside of the linear element (1).

6. Surgical instrument according to Claim 5, **characterized in that** the connection pin (19) has a threaded portion (18) with which it can be fitted into a threaded bore (17) in the limit stop.

7. Surgical instrument according to Claim 5 or 6, **characterized in that** the slide button (22) is guided in a channel (23) in the linear element (1).

8. Surgical instrument according to at least one of Claims 1 to 7, **characterized in that** the linear element is a shaft (1), and the insert or attachment is a slide (7) of a sliding shaft instrument, said shaft (1) having a longitudinal groove (11.1, 11.2; 12.1, 12.2) as guide.

9. Surgical instrument according to at least one of Claims 1 to 8, **characterized in that** a pressure pin (14) is provided as limit stop.

## Revendications

1. Instrument chirurgical doté d'un élément linéaire (1) et d'un insert ou d'une applique (7) disposés à coulissement et de manière libérable dans ou sur l'élément linéaire (1), l'insert ou l'applique (7) et/ou un élément de guidage (8.1, 8.2) qui leur est associé étant guidés par rapport à l'élément linéaire (1) sur au moins une piste de guidage (11.1, 11.2; 12.1, 12.2) et une butée (14) pouvant être déplacée dans ou hors de la piste de guidage (11.2, 12.2) derrière l'insert ou l'applique (7) et/ou l'élément de guidage (8.2),
**caractérisé en ce que**
la butée (14) est guidée obliquement sous un angle obtus vers la piste de guidage (11.2), de préférence du bas vers le haut.

2. Instrument chirurgical selon la revendication 1, **caractérisé en ce que** la butée (14) est guidée dans un trou aveugle ménagé dans l'élément linéaire (1).

3. Instrument chirurgical selon les revendications 1 ou 2, **caractérisé en ce que** la butée (14) s'appuie contre un accumulateur de force (16).

4. Instrument chirurgical selon la revendication 3, **caractérisé en ce que** l'accumulateur de force est un ressort hélicoïdal (16).

5. Instrument chirurgical selon au moins l'une des revendications 1 à 4, **caractérisé en ce que** la butée (14) est reliée par l'intermédiaire d'une tige de liaison (19) à un bouton coulissant (22) situé à l'extérieur de l'élément linéaire (1).

6. Instrument chirurgical selon la revendication 5, **caractérisé en ce que** la tige de liaison (19) présente une partie filetée (18) par laquelle elle peut être insérée dans un alésage fileté (17) ménagé dans la butée.

7. Instrument chirurgical selon les revendications 5 ou 6, **caractérisé en ce que** le bouton coulissant (22) est guidé dans une rainure (23) ménagée dans l'élément linéaire (1).

8. Instrument chirurgical selon au moins l'une des revendications 1 à 7, **caractérisé en ce que** l'élément linéaire est une tige (1) et l'insert ou l'applique est un coulisseau (7) d'un instrument à tige coulissante, la tige (1) présentant comme guide une rainure longitudinale (11.1, 11.2; 12.1, 12.2).

9. Instrument chirurgical selon au moins l'une des revendications 1 à 8, **caractérisé en ce qu'**une tige de poussée (14) est prévue comme butée.
